# FASCICULE DE BREVET EUROPEEN

(11) **EP 0 456 279 B1**
(45) Date de publication et mention de la délivrance du brevet: **03.01.1996**
(21) Numéro de dépôt: 91111702.6
(22) Date de dépôt: 28.06.1988
(51) Int. Cl.: C07D 221/04, A61K 31/435

(54) **Dérivés condensés de la pipéridine, leur procédé de préparation et les intermédiaires de préparation, leur application comme médicaments et compositions pharmaceutiques les renfermant**
Kondensierte Piperidinderivate, Zwischenprodukte und Verfahren zu ihrer Herstellung, ihre Verwendung als Arzneimittel und pharmazeutische Zusammensetzungen davon
Condensed piperidine derivatives, intermediates and process for their preparation, their use as medicaments and pharmaceutical compositions containing them

(30) Priorité: 03.07.1987 FR 8709449
(43) Date de publication de la demande: 13.11.1991
(62) Demande divisionnaire de: 88401650.2
(73) Titulaire: ROUSSEL UCLAF, F-93230 Romainville (FR)
(72) Inventeur: Clemence, François, F-75009 Paris (FR); Frechet, Daniel, F-75015 Paris (FR); Fortin, Michel, F-75018 Paris (FR)
(74) Mandataire: Vieillefosse, Jean-Claude

(56) Documents cités:
- EP-A- 0 002 937
- EP-A- 0 146 297
- FR-A- 2 592 879

## Description

L'invention concerne des dérivés de la tétrahydropyridine, leur procédé et les intermédiaires de préparation, leur application comme médicaments et les compositions pharmaceutiques les renfermant.

L'invention concerne des composés de formule (IB) : 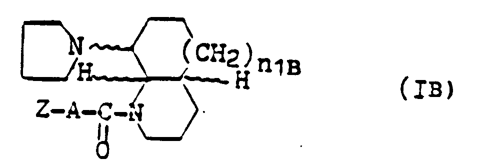
dans laquelle n₁B représente 2, A représente une chaîne 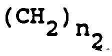
dans laquelle n₂ représente un nombre de 0 à 5 ou A représente une chaîne alcoylène substituée par un radical alcoyle renfermant au total 2 à 8 atomes de carbone, Z représente un radical phényle, un radical naphtyle, un radical indényle, un radical thiazolyle, pyridinyle, oxazolyle, isoxazolyle, imidazolyle, thiényle, furyle ou pyrimidinyle ou un radical indolyle, quinolyle, benzofuranyle, benzo[b]thiényle, benzimidazolyle, benzoxazolyle ou benzothiazolyle, tous ces radicaux étant éventuellement substitués par un ou plusieurs radicaux identiques ou différents choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, lesdits composés de formule (IB) pouvant être dans toutes les formes énantiomères et diastéréoisomères possibles et sous forme de sels d'addition avec les acides ou de sels d'ammonium quaternaire.

Lorsque A représente une chaîne 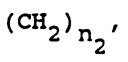
n₂ est de préférence égal à O ou 1.

Lorsque A représente une chaîne alcoylène substituée par un radical alcoyle, par alcoyle on entend de préférence méthyle ou éthyle et A est alors de préférence un radical 1,1-éthanediyl, 1-méthyl 1,2-éthanediyl, 1-méthyl ou 2-méthyl 1,3-propanediyl, 1-éthyl 1,2-éthanediyl.

Par substituant alcoyle, alcoxy ou halogène, on entend de préférence méthyle, éthyle, propyle ou butyle linéaire ou ramifié, méthoxy, éthoxy, propoxy ou butoxy linéaire ou ramifié, fluoro, chloro, bromo ou iodo.

Dans les valeurs monoalkyl et dialkylamino, les radicaux alkyles sont préférentiellement les radicaux méthyle ou éthyle.

Par ailleurs, un composé de formule (IB) peut exister sous la forme de quatre racémates, ou paire d'énantiomères. Les énantiomères de chaque paire peuvent être séparés par des procédés classiques. L'invention couvre donc toutes les formes énantiomères et diastéréoisomères des composés de formule (IB).

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide méthane sulfonique et arylsulfoniques, tels que l'acide benzène sulfonique.

L'invention concerne aussi des composés de formule (IB) sous forme de sels d'ammonium quaternaire.

Par sels d'ammonium quaternaire, on entend les composés de formule (IB) quaternisés par des produits de type R-Y, R étant un radical alcoyle ayant de 1 à 4 atomes de carbone tel qu'un radical méthyle, éthyle, n-propyle ou isopropyle et Y un anion halogénure, par exemple, un chlorure, un bromure, un iodure.

L'invention concerne notamment des composés de formule IB dans laquelle A représente une chaîne 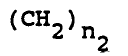
où n₂ est égal à O ou 1, ou une chaîne 1,1-éthanediyl, ainsi que leurs sels d'addition avec les acides et leurs sels d'ammonium quaternaire.

L'invention a aussi plus particulièrement pour objet des composés de formule IB dans laquelle Z représente un radical phényle, naphtyle, pyridinyle, thiényle, indolyle, benzo[b]thiényle, éventuellement substitué par un ou plusieurs substituants identiques ou différents, ainsi que leurs sels d'addition avec les acides et leurs sels d'ammonium quaternaire.

L'invention concerne aussi plus particulièrement des composés de formule (IB) dans laquelle Z représente un radical phényle substitué par un ou plusieurs substituants choisis dans le groupe constitué par les atomes d'halogène, le radical trifluorométhyle ou nitro ou Z représente un radical naphtyle ou benzo[b]thiényle, ainsi que leurs sels d'addition avec les acides et leurs sels d'ammonium quaternaire.

L'invention a aussi pour objet un procédé de préparation des composés de formule (IB) caractérisé en ce que l'on condense un composé de formule (II) : 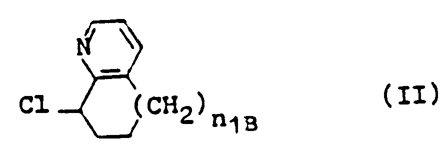
dans laquelle n₁B est 2 avec la pyrrolidine pour obtenir un composé de formule (III) : 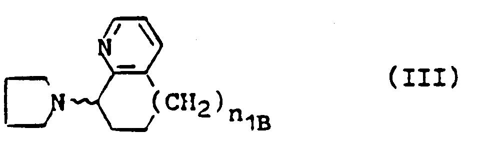
que l'on réduit pour obtenir un composé de formule (IV), dont on sépare éventuellement les différents isomères : 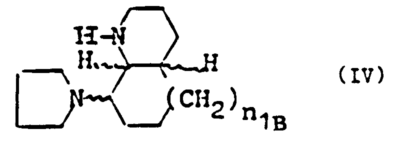
que l'on condense avec un composé de formule V ou un dérivé fonctionnel de ce composé : 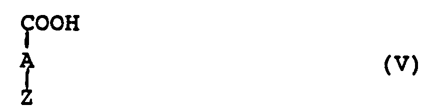
dans laquelle A et Z ont les significations indiquées précédemment pour obtenir un composé de formule (IB) sous toutes les formes énantiomères et diastéréoisomères possibles, que l'on traite, si désiré, avec un acide minéral ou organique pour obtenir un sel ou avec un halogénure d'alcoyle pour obtenir un sel d'ammonium quaternaire.

Dans un mode de réalisation préférée du procédé de l'invention :
- La condensation du produit de formule II avec la pyrrolidine en milieu aqueux s'effectue à une température variant de 20°C à 120°C.
- La réduction du composé de formule III est une hydrogénation catalytique. Le catalyseur utilisé est de préférence l'oxyde de platine.

Cette réduction est réalisée en présence d'un acide tel que l'acide chlorhydrique.
- L'hydrogénation des produits de formule III conduit aux différents isomères à jonction de cycle cis ou trans, chacun des deux isomères cis ou trans pouvant porter le groupement pyrrolidinyl dans une orientation alpha ou béta.
- L'activation de la fonction carboxyle du composé de formule V, pour réaliser la condensation avec le composé de formule IV, s'effectue en présence de carbonyldiimidazole ou de dicyclohexylcarbodiimide. On peut également activer l'acide de formule IV sous la forme d'un chlorure d'acide ou d'un anhydride mixte.

Ces différents isomères sont séparés par chromatographie.

Les composés de formule IB tels que définis ci-dessus ainsi que leurs sels d'addition avec les acides présentent d'intéressantes propriétés pharmacologiques. Ils présentent, en particulier, une forte affinité pour les récepteurs opiacés et notamment pour les récepteurs K et sont doués de propriétés analgésiques centrales.

Ils sont doués également de propriétés diurétiques, de propriétés anti-arythmiques, anti ischémiques cérébrales et hypotensives.

Ces propriétés justifient leur application en thérapeutique et l'invention a également pour objet à titre de médicaments, les produits tels que définis par la formule (IB) ci-dessus ainsi que leurs sels d'addition avec les acides pharmaceutiquement acceptables et leurs sels d'ammonium quaternaire.

Les médicaments, objet de l'invention, permettent notamment de soulager une douleur quelle qu'en soit l'origine, par exemple une douleur de nature musculaire, articulaire ou nerveuse.

Ils peuvent être aussi utilisés dans le traitement des douleurs dentaires, des migraines, du zona dans le traitement des douleurs intenses, en particulier rebelles aux antalgiques périphériques, par exemple au cours des processus néoplasiques, dans le traitement des pancréatites, coliques néphrétiques ou biliaires, dans le traitement des douleurs post-opératoires et post-traumatiques.

La posologie varie notamment en fonction de la voie d'administration, de l'affection traitée et du sujet en cause.

Par exemple, chez l'adulte, elle peut varier entre 20 et 400 mg de principe actif par jour, par voie orale et entre 5 et 100 mg par jour, par voie parentérale.

Les médicaments, objet de l'invention, trouvent aussi leur emploi, dans le traitement des arythmies.

La dose usuelle, variable selon le dérivé utilisé, le sujet et l'affection en cause, peut être par exemple de 50 mg à 1 g par jour.
Par voie orale, le principe actif peut être administré à la dose quotidienne de 200 mg à 800 mg, par exemple pour le traitement des arythmies ventriculaires, supraventriculaires et jonctionnelles, soit environ de 3 mg à 12 mg par kilogramme de poids corporel.

Les médicaments, objet de l'invention, peuvent aussi être utilisés dans le traitement des syndromes oedémateux de l'insuffisance cardiaque, de certaines obésités, des cirrhoses, dans le traitement des oedèmes sévères et réfractaires, en particulier ceux de l'insuffisance cardiaque congestive et dans le traitement au long cours de l'hypertension artérielle.

La dose quotidienne de principe actif est variable. Elle peut être par exemple de 60 à 100 mg par jour par voie orale.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule II utilisés comme produits de départ dans le procédé de l'invention sont préparés par chloruration des produits hydroxylés correspondants.

Ces produits hydroxylés sont décrits dans J. Chem. Soc. Perkin Trans(1) 1973(9) 968 à 972 et dans J. Am. Chem. Soc.80, 6254 (1958).

Les composés de formules II, III et IV sont des produits chimiques intermédiaires.

L'invention a donc pour objet ces produits à titre de produits industriels.

Les exemples suivants illustrent l'invention sans toutefois la limiter.

### Exemple de référence : Chlorhydrate de [4aRS (4a-alpha, 7a-alpha)] (^{±}) 1-[(3,4- dichlorophényl) acétyl] octahydro 7-(1-pyrrolidinyl) 1H-1-pyrindine (isomère cis I)

### Stade A : 7-(1-pyrrolidinyl) 6,7-dihydro 5H-1-pyrindine.

On dissout 5 g de chlorhydrate de 7-chloro 6,7-dihydro 5H- 1-pyrindine dans 10 cm3 d'eau, ajoute 8,8 cm3 de pyrrolidine et agite pendant 2 heures et demie à température ambiante. On extrait au chlorure de méthylène, lave à l'eau, sèche et concentre à sec sous pression réduite. On reprend le résidu à l'acétate d'éthyle, traite au charbon actif, filtre et amène à sec sous pression réduite. On chromatographie le résidu sur silice, élue par un mélange acétate d'éthyle triéthylamine (95-5) et récupère 4,71 g de produit attendu.
Spectre RMN CDCl₃ ppm :
8,41 ( d, J = 5 Hz) H₂ pyrindine
7,06 (dd, J = 5 et 5 Hz) H₃ pyrindine
7,90 ( d, J = 8 Hz) H₄ pyrindine
2,11 à 3,06 H₅, H₆ pyrindine
4,06 (dd, J = 5,5 et 8 Hz) H₇ pyrindine

### Préparation du chlorhydrate de 7-chloro 6,7-dihydro 5H-1-pyrindine

On met en solution 10 g de 7-hydroxy 6,7-dihydro 5H-1-pyrindine dans 100 cm3 de chlorure de méthylène, refroidit à 0°C pour ajouter 20 cm3 de chlorure de thionyle. On agite 30 minutes à 0°C, ajoute 250 cm3 d'éther et agite encore 30 minutes à 0°C. On essore, lave à l'éther, sèche sous pression réduite à 80°C et obtient 13,60 g de produit. On dissout ce dernier à chaud dans 400 cm3 de chlorure de méthylène, traite au charbon actif, filtre et concentre à environ 100 cm3. Après refroidissement à température ambiante, on ajoute 200 cm3 d'éther, laisse cristalliser à température ambiante pendant 2 heures, essore, lave à l'éther et obtient 10,81 g de produit attendu F = 130-135°C.

### Stade B : (±) octahydro 7-(1-pyrrolidinyl) 1H-1-pyrindine isomères cis et trans.

On hydrogène 6,4 g du produit obtenu au stade A dans 64 cm3 de méthanol, 6,4 cm3 d'acide chlorhydrique concentré en présence d'oxyde de platine pendant 5 heures sous 17OO mbars. On filtre, évapore à sec le solvant à 40°C maximum sous pression réduite, reprend le résidu par 20 cm3 d'eau, alcalinise par environ 10 cm3 d'une solution de soude à 32 %, extrait au chlorure de méthylène, lave à l'eau, sèche et amène à sec sous pression réduite. On chromatographie le résidu sur silice, élue par un mélange : acétate d'éthyle-méthanol-triéthylamine (80-15-5) et sépare :
2,2 g isomère (cis II) rf 0,35
0,80 g isomère (trans I) rf 0,20
0,15 g isomère (cis I) rf 0,15
Spectre RMN (CDCl₃) ppm :
Isomère cis I :

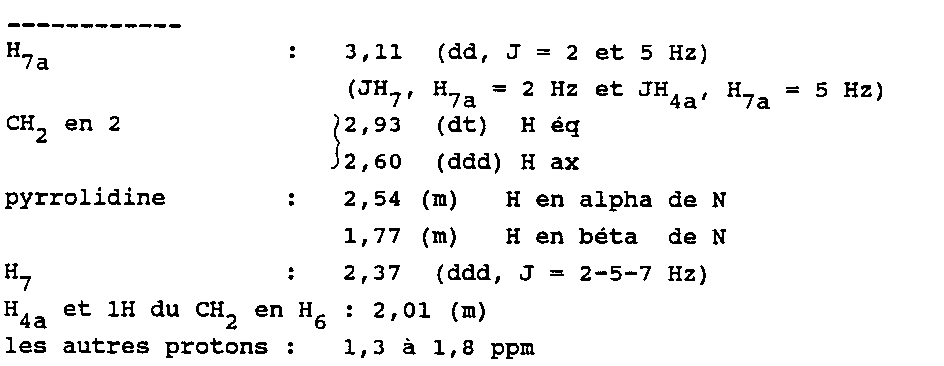

Isomère cis II :

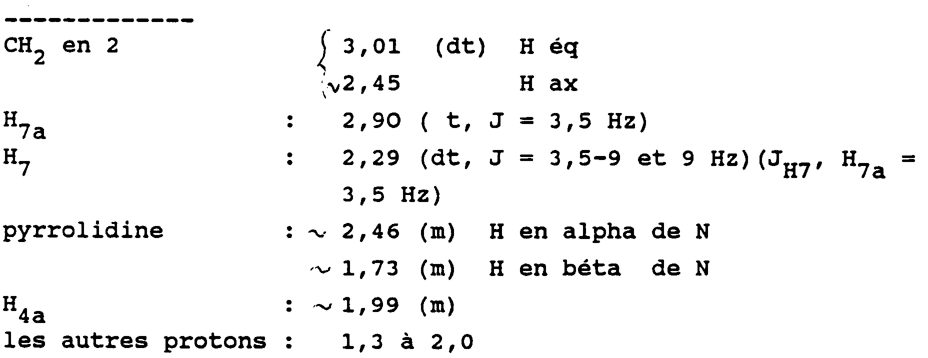

Isomère trans I :

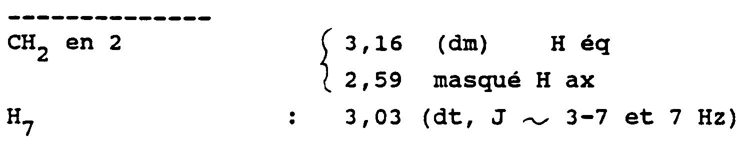

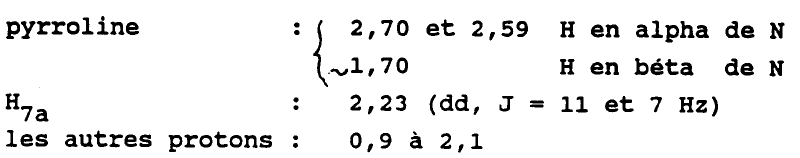

### Stade C : Chlorhydrate de [4aRS (4a-alpha, 7a-alpha)] (±) 1-[(3,4-dichlorophényl)acétyl] octahydro 7-(1-pyrrolidinyl) 1H-1-pyrindine (isomère cis I)

On met en solution 1,43 g d'acide dichlorophényl acétique dans 9,7 cm3 de tétrahydrofuranne, ajoute 1,14 g de carbonyl diimidazole et agite 1 heure à température ambiante. On ajoute 0,972 g d'isomère cis I obtenu au stade B dissous dans 9,7 cm3 de tétrahydrofuranne et agite 5 heures à température ambiante. On amène à sec sous pression réduite en chauffant à 40°C. On extrait le résidu à l'éther, lave la phase organique avec une solution de bicarbonate de sodium, puis à l'eau, sèche et amène à sec. On chromatographie sur silice le résidu, élue par un mélange acétate d'éthyle, méthanol, triéthylamine (80-15-5) et récupère 1,66 g de produit attendu sous forme de base.

On dissout 1,6 g de ce dernier dans 5 cm3 d'acétate d'éthyle, ajoute un excès d'une solution d'acide chlorhydrique dans l'acétate d'éthyle, laisse cristaller puis essore. On obtient 1,36 g de chlorhydrate attendu F = 228°C après recristallisation dans l'acétate d'éthyle.
Spectre RMN (CDCl₃) ppm :

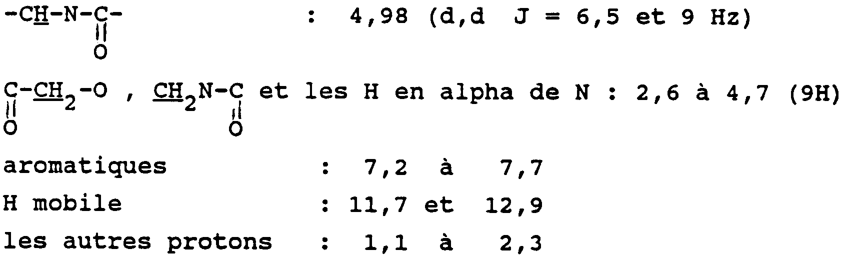

### Exemple 1 : Chlorhydrate de [4a-alpha,9béta,9a-alpha] (+) décahydro 1-[(3,4-dichlorophényl) acétyl] 9-(1-pyrrolidinyl) 1H-cyclohepta [b] pyridine

### Stade A : 9-(1-pyrrolidinyl) 6,7,8,9-tétrahydro 5H-cyclohepta [b] pyridine.

On dissout 5 g de chlorydrate de 9-chloro 6,7,8,9-tétrahydro 5H-cyclohepta [b] pyridine dans 12,5 cm3 d'eau et ajoute 8 cm3 de pyrrolidine. On porte à reflux sous vive agitation pendant 5 heures, évapore sous pression réduite l'excès de pyrrolidine, ajoute 25 cm3 d'une solution saturée de bicarbonate de sodium, puis 50 cm3 d'eau puis extrait au chlorure de méthylène. On lave à l'eau les phases organigues réunies, sèche et concentre à sec sous pression réduite. On chromatographie le résidu sur silice, élue par un mélange chlorure de méthylène-méthanol (9-1) et recueille 2,25g de produit attendu (Rf 0,20). On cristallise 2,25 g de produit dans l'éther de pétrole (eb 40°-70°C) pour obtenir 2 g de produit attendu F ≃ 62°C.

### Préparation du chlorhydrate de 9-chloro 6,7,8,9-tétrahydro 5H-cyclohepta [b] pyridine

On porte à reflux pendant 1 heure, 26 g de chlorhydrate de 9-hydroxy 6,7,8,9-tétrahydro 5 H-cyclohepta [b] pyridine dans 520 cm3 de chlorure de méthylène et 130 cm3 de chlorure de thionyle. On évapore presqu'à sec sous pression réduite à ∼ 45°C, ajoute 26 cm3 d'acétate d'éthyle, amorce la cristallisation, essore et obtient 24 g de produit. On dissout ce dernier dans 40 cm3 de méthanol, ajoute 100 cm3 de méthyl éthylcétone, concentre à 80 cm3, filtre et concentre à 30 cm3. On amorce la cristallisation et essore 20,4 g de produit attendu F ≃ 150°C.

### Stade B : [4a-alpha, 9béta, 9a-alpha] (±) décahydro, 9-(1-pyrrolidinyl) 1H-cyclohepta [b] pyridine.

On opère comme au stade B de l'exemple de référence pour hydrogéner durant 3 heures 4,2 g de produit obtenu ci-dessus (F ∼ 62°C) et obtenir 4 g de produit attendu F ≃ 30-35°C.

### Stade C : Chlorhydrate de [4a-alpha, 9béta, 9a-alpha)](±) décahydro 1-[3,4-dichlorophényl) acétyl] 9-(1-pyrrolidinyl) 1h-cyclohepta [b] pyridine.

On opère comme au stade C de l'exemple de référence à partir de 2,22 g de produit obtenu précédemment, chromatographie le résidu sur silice, élue par un mélange chlorure de méthylène-méthanol (9-1) et obtient 1,75 g de produit attendu sous forme de base. On dissout ce dernier dans 10 cm3 d'acétate d'éthyle, laisse cristalliser, essore 1,65 g de chlorhydrate attendu F ≃ 190°C. On reprend celui-ci à chaud dans 20 cm3 d'éthanol, filtre, ramène à 20°C et ajoute 30 cm3 d'éther. On amorce la cristallisation, laisse cristalliser 16 heures, essore et obtient 1,2 g de produit attendu. F ≃ 110°C, puis ≃ 190°C.
Spectre UV (Ethanol) :
Inf 220 nm E₁¹ 303 ε = 13.500
Inf 222 nm E₁¹ 274
Inf 227 nm E₁¹ 218
Inf 260 nm E₁¹ 4
Inf 268 nm E₁¹ 6
Inf 272 nm E₁¹ 8,5 ε = 400
Inf 281 nm E₁¹ 8

### Exemple 2 : Chlorhydrate de [4a-alpha,(9alpha,9a-aplha)] (+) décahydro 1-[(3,4-dichlorophényl) acétyl] 9-(1-pyridinyl) 1H-cyclohepta [b] pyridine

En opérant comme au stade C de l'exemple 1 à partir du [4a-alpha, 9-alpha, 9a-alpha) (±) décahydro 9-(1-pyrrolidinyl) 1H-cyclohepta [b] pyridine (F + 65°C) lui-même obtenu par chromatographie après la réduction du stade B, on a obtenu le produit attendu. F = 150°C puis 250°C.

### Exemple 3 :

On a préparé des comprimés répondant à la formule suivante :

| | |
|---|---|
| - produit de l'exemple 1 | 200 mg |
| - excipient q.s.p. | 800 mg |

(détail de l'excipient : lactose, talc, amidon, stéarate de magnésium).

### Exemple 4 :

On a préparé un soluté injectable (voie intra-musculaire) répondant à la formule suivante :

| | |
|---|---|
| - produit de l'exemple 1 | 50 mg |
| - solvant stérile q.s.p | 5 cm3 |

### ETUDE PHARMACOLOGIQUE

### Action antiarythmique chez le rat :

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse.

Cinq minutes après l'administration du produit, on perfuse la veine jugulaire des rats avec 10 ug/mn sous 0,2 ml d'une solution d'aconitine et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que certains des produits de la présente demande sont doués de bonnes propriétés antiarythmiques.

| Produit de l'exemple | Dose mg/kg | Pourcentage d'allongement du temps |
|---|---|---|
| 1 | 2,5 | +65 % |
| | 1 | +37 % |

## Revendications

1. Composés de formule (IB) : 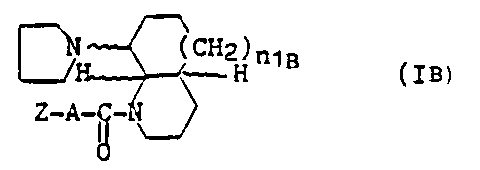 dans laquelle n_{1B} représente 2, A représente une chaîne 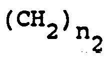 dans laquelle n₂ représente un nombre de 0 à 5 ou A représente une chaîne alcoylène substituée par un radical alcoyle renfermant au total 2 à 8 atomes de carbone, Z représente un radical phényle, un radical naphtyle, un radical indényle, un radical thiazolyle, pyridinyle, oxazolyle, isoxazolyle, imidazolyle, thiényle, furyle ou pyrimidinyle ou un radical indolyle, quinolyle, benzofuranyle, benzo[b]thiényle, benzimidazolyle, benzoxazolyle ou benzothiazolyle, tous ces radicaux étant éventuellement substitués par un ou plusieurs radicaux identiques ou différents choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, lesdits composés de formule (IB) pouvant être dans toutes les formes énantiomères et diastéréoisomères possibles et sous forme de sels d'addition avec les acides ou de sels d'ammonium quaternaire.

2. Composés de formule (IB) telle que définie à la revendication 1, dans laquelle A représente une chaîne (CH₂)ₙ₂ où n₂ est égal à O ou à 1, ou une chaîne 1,1-éthanediyl, ainsi que leurs sels d'addition avec les acides et leurs sels d'ammonium quaternaire.

3. Composés de formule (IB) telle que définie à la revendication 1 ou 2, dans laquelle Z représente un radical phényle, naphtyle, pyridinyle, thiényle, indolyle, benzo[b]thiényle, éventuellement substitué par un ou plusieurs substituants identiques ou différents, ainsi que leurs sels d'addition avec les acides et leurs sels d'ammonium quaternaire.

4. Composés de formule (IB) telle que définie à l'une quelconque des revendications 1 à 3, dans laquelle Z représente un radical phényle substitué par un ou plusieurs substituants choisis dans le groupe constitué par les atomes d'halogène, le radical trifluorométhyle ou nitro ou Z représente un radical naphtyle ou benzo[b]thiényle, ainsi que leurs sels d'addition avec les acides et leurs sels d'ammonium quaternaire.

5. L'un quelconque des composés de formule (IB) telle que définie à la revendication 1, dont les noms suivent :
- Le [4a-alpha, 9béta, 9a-alpha] (±) décahydro 1-[(3,4-dichlorophényl) acétyl] 9-(1-pyrrolidinyl) 1H-cyclohepta [b] pyridine,
- le [4a-alpha, 9alpha, 9a-alpha] (±) décahydro 1-[(3,4-dichlorophényl) acétyl] 9-(1-pyrrolidinyl) 1H-cyclohepta [b] pyridine, ainsi que leurs sels d'addition avec les acides et leurs sels d'ammonium quaternaire.

6. Procédé de préparation des composés de formule (IB) tels que définis aux revendications 1 à 5, caractérisé en ce que l'on condense un composé de formule (II) : 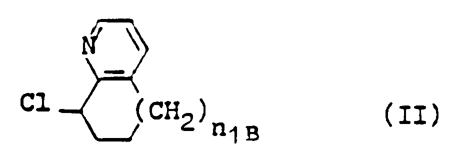 dans laquelle n₁B est 2 avec la pyrrolidine pour obtenir un composé de formule (III) : 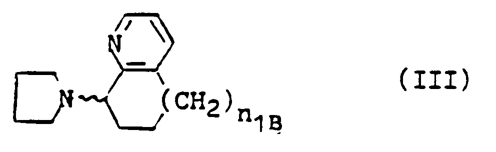 que l'on réduit pour obtenir un composé de formule (IV), dont on sépare éventuellement les différents isomères : 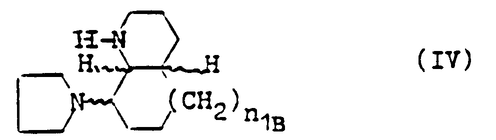 que l'on condense avec un composé de formule V ou un dérivé fonctionnel de ce composé : 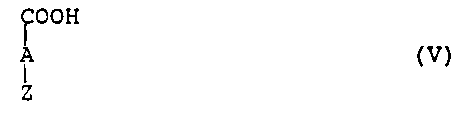 dans laquelle A et Z ont les significations indiquées précédemment pour obtenir un composé de formule (IB) sous toutes les formes énantiomères et diastéréoisomères possibles, que l'on traite, si désiré, avec un acide minéral ou organique pour obtenir un sel ou avec un halogénure d'alcoyle pour obtenir un sel d'ammonium quaternaire.

7. A titre de médicaments, les produits de formule (IB) tels que définis aux revendications 1 à 4.

8. A titre de médicaments, les produits de la revendication 5.

9. Les compositions pharmaceutiques contenant à titre de principe actif, l'un au moins des médicaments, tels que définis aux revendications 7 et 8.

10. A titre de produit industriel, le composé de formule II : 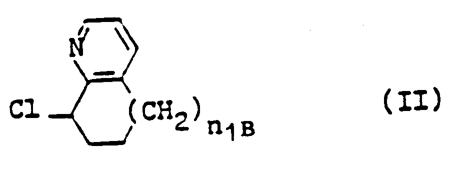 dans laquelle n₁B est 2.

11. A titre de produit industriel, le composé de formule III : 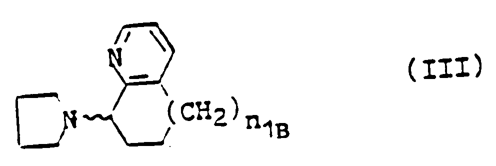 dans laquelle n₁B est 2.

12. A titre de produit industriel, le composé de formule IV : 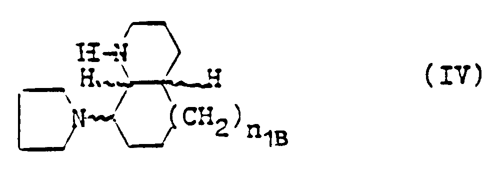 dans laquelle n₁B est 2.

## Claims

1. The compounds of formula (IB): 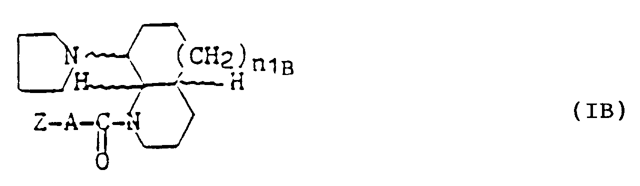 in which n_{1B} represents 2, A represents a (CH₂)ₙ₂ chain in which n₂ represents a number from 0 to 5 or A represents an alkylene chain substituted by an alkyl radical containing at most 2 to 8 carbon atoms, Z represents one of the following radicals: phenyl, naphthyl, indenyl, thiazolyl, pyridinyl, oxazolyl, isoxazolyl, imidazolyl, thienyl, furyl or pyrimidinyl, or indolyl, quinolyl, benzofuranyl, benzo[b]thienyl, benzimidazolyl, benzoxazolyl or benzothiazolyl, all these radicals being optionally substituted by one or more identical or different radicals chosen from the group constituted by alkyl radicals containing 1 to 5 carbon atoms, alkoxy radicals containing 1 to 5 carbon atoms, halogen atoms, hydroxyl, trifluoromethyl, nitro, amino, monoalkyl- or dialkylamino radicals the alkyl radicals of which contain 1 to 5 carbon atoms, the said compounds of formula (IB) can be in all possible enantiomeric and diastereoisomeric forms and in the form of addition salts with acids or quaternary ammonium salts.

2. The compounds of formula (IB) as defined in claim 1, in which A represents a (CH₂)ₙ₂ chain where n₂ is equal to 0 or 1, or a 1,1-ethanediyl chain, as well as their addition salts with acids and their quaternary ammonium salts.

3. The compounds of formula (IB) as defined in claim 1 or 2, in which Z represents a phenyl, naphthyl, pyridinyl, thienyl, indolyl, benzo[b]thienyl radical, optionally substituted by one or more identical or different substituents, as well as their addition salts with acids and their quaternary ammonium salts.

4. The compounds of formula (IB) as defined in any one of claims 1 to 3, in which Z represents a phenyl radical substituted by one or more substituents chosen from the group constituted by halogen atoms, the trifluoromethyl or nitro radical or Z represents a naphthyl or benzo[b]thienyl radical, as well as their addition salts with acids and their quaternary ammonium salts.

5. Any one of the compounds of formula (IB) as defined in claim 1 of which the names follow:
- [4a-alpha, 9-beta, 9a-alpha)] (±) decahydro 1-[(3,4-dichlorophenyl) acetyl] 9-(1-pyrrolidinyl) 1H-cyclohepta [b] pyrindine,
- [4a-alpha, 9alpha, 9a-alpha) (±) decahydro 1-[(3,4-dichlorophenyl) acetyl] 9-(1-pyrrolidinyl) 1H-cyclohepta [b] pyrindine, as well as their addition salts with acids and their quaternary ammonium salts.

6. Preparation process for the compounds of formula (IB) as defined in claims 1 to 5, characterized in that a compound of formula (II): 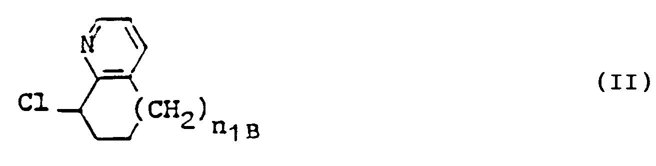 in which n₁B is 2, is condensed with pyrrolidine in order to obtain a compound of formula (III): 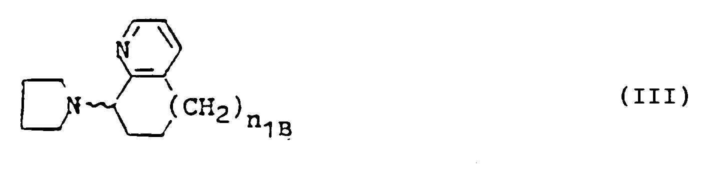 which is reduced in order to obtain a compound of formula (IV), the different isomers of which are optionally separated: 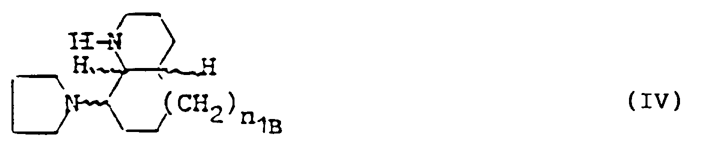 which is condensed with a compound of formula (V) or a functional derivative of this compound: 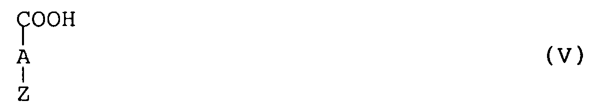 in which A and Z have the meanings indicated previously in order to obtain a compound of formula (IB) in all possible enantiomeric and diastereoisomeric forms, which is treated, if desired, with a mineral or organic acid in order to obtain a salt or with an alkyl halide in order to obtain a quaternary ammonium salt.

7. As medicaments, the products of formula (IB) as defined in claims 1 to 4.

8. As medicaments, the products of claim 5.

9. The pharmaceutical compositions containing as active ingredient, at least one of the medicaments, as defined in claims 7 and 8.

10. As an industrial product, the compound of formula (II): 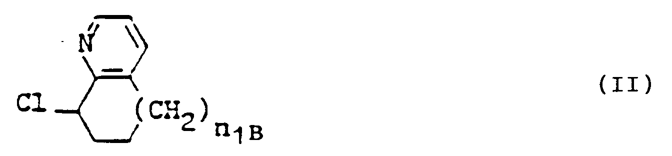 in which n₁B is 2.

11. As an industrial product, the compound of formula (III): 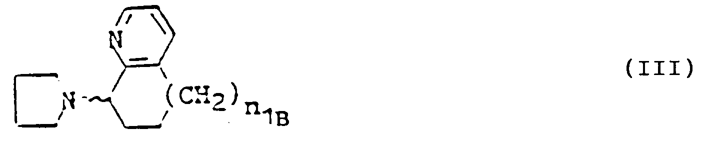 in which n₁B is 2.

12. As an industrial product, the compound of formula (IV): 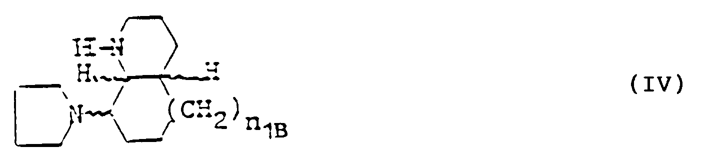 in which n₁B is 2

## Patentansprüche

1. Verbindungen der Formel (IB): 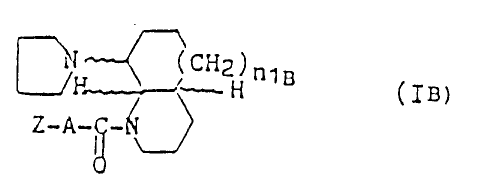 in der n_{1B} für 2 steht, A eine Kette (CH₂)ₙ₂ darstellt, in der n₂ für eine Zahl von 0 bis 5 steht, oder A eine Alkylenkette darstellt, substituiert durch einen Alkylrest, insgesamt 2 bis 8 Kohlenstoffatome einschließend, Z einen Phenylrest, einen Naphthylrest, einen Indenylrest, einen Thiazolyl-, Pyridinyl-, Oxazolyl-, Isoxazolyl-, Imidazolyl-, Thienyl-, Furyl- oder Pyrimidinylrest oder einen Indolyl-, Chinolyl-, Benzofuranyl-, Benzo[b]thienyl-, Benzimidazolyl-, Benzoxazolyl- oder Benzothiazolyl-Rest darstellt, wobei alle diese Reste gegebenenfalls durch einen oder mehrere identische oder verschiedene Reste substituiert sind, die aus der Gruppe ausgewählt sind, die aus Alkylresten mit 1 bis 5 Kohlenstoffatomen, Alkoxyresten mit 1 bis 5 Kohlenstoffatomen, Halogenatomen, Hydroxyl-, Trifluormethyl, Nitro-, Amino-, Monoalkyl- oder Dialkylamino-Resten, deren Alkylreste 1 bis 5 Kohlenstoffatome einschließen, besteht, wobei die Verbindungen der Formel (IB) in allen ihren möglichen enantiomeren und diastereomeren Formen und in Form von Additionssalzen mit Säuren oder quaternären Ammoniumsalzen vorliegen können.

2. Verbindungen der Formel (IB), wie in Anspruch 1 definiert, in der A eine Kette (CH₂)ₙ₂, worin n₂ gleich 0 oder gleich 1 ist, oder eine 1,1-Ethandiyl-Kette darstellt, sowie ihre Additionssalze mit Säuren und ihre quaternären Ammoniumsalze.

3. Verbindungen der Formel (IB), wie in Anspruch 1 oder 2 definiert, in der Z einen Phenyl-, Naphthyl-, Pyridinyl-, Thienyl-, Indolyl-, Benzo[b]thienyl-Rest darstellt, der gegebenenfalls durch einen oder mehrere identische oder verschiedene Substituenten substituiert ist, sowie ihre Additionssalze mit Säuren und ihre quaternären Ammoniumsalze.

4. Verbindungen der Formel (IB), wie in irgendeinem der Ansprüche 1 bis 3 definiert, in der Z einen Phenylrest darstellt, der durch einen oder mehrere Substituenten substituiert ist, die aus der Gruppe ausgewählt sind, die aus Halogenatomen, dem Trifluormethyl- oder Nitrorest besteht, oder Z einen Naphthyl- oder Benzo[b]thienyl-Rest darstellt, sowie ihre Additionssalze mit Säuren und ihre quaternären Ammoniumsalze.

5. Irgendeine der Verbindungen der Formel (IB), wie in Anspruch 1 definiert, deren Namen folgen:
- [4a-alpha, 9-beta, 9a-alpha]-(±)-Decahydro-1-[(3,4-dichlorphenyl)acetyl]-9-(1-pyrrolidinyl)-1H-cyclohepta[b]pyridin,
- [4a-alpha, 9-alpha, 9a-alpha]-(±)-Decahydro-1-[(3,4-dichlorphenyl)acetyl]-9-(1-pyrrolidinyl)-1H-cyclohepta[b]pyridin sowie ihre Additionssalze mit Säuren und ihre quaternären Ammoniumsalze.

6. Verfahren zur Herstellung von Verbindungen der Formel (IB), wie in den Ansprüchen 1 bis 5 definiert, dadurch gekennzeichnet, daß man eine Verbindung der Formel (II): 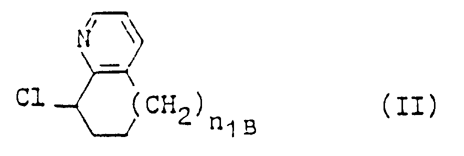 in der n₁B 2 ist, mit Pyrrolidin kondensiert, um eine Verbindung der Formel (III): 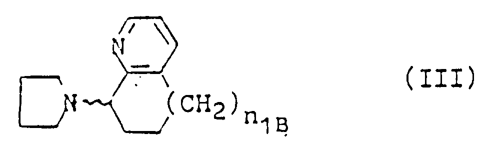 zu erhalten, die man reduziert, um eine Verbindung der Formel (IV) zu erhalten, bei der man gegebenenfalls die verschiedenen Isomeren trennt: 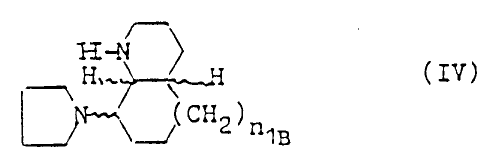 welche man mit einer Verbindung der Formel (V) oder einem funktionellen Derivat dieser Verbindung kondensiert: 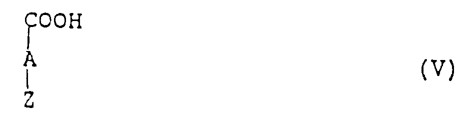 in der A und Z die vorstehend angegebenen Bedeutungen aufweisen, um eine Verbindung der Formel (IB) in allen möglichen enantiomeren und diastereomeren Formen zu erhalten, die man, falls gewünscht, mit einer Mineral- oder organischen Säure zum Erhalt eines Salzes oder mit einem Alkylhalogenid zum Erhalt eines quaternären Ammoniumsalzes behandelt.

7. Die Produkte der Formel (IB), wie in den Ansprüchen 1 bis 4 definiert, als Arzneistoffe.

8. Die Produkte des Anspruchs 5 als Arzneistoffe.

9. Pharmazeutische Zusammensetzungen, die als Wirkstoff mindestens einen der Arzneistoffe, wie in den Ansprüchen 7 und 8 definiert, enthalten.

10. Verbindung der Formel II: 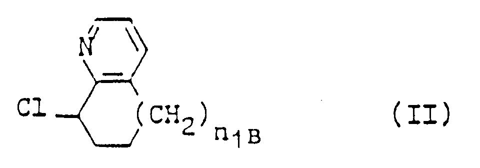 in der n₁B 2 ist, als industrielles Produkt.

11. Verbindung der Formel III: 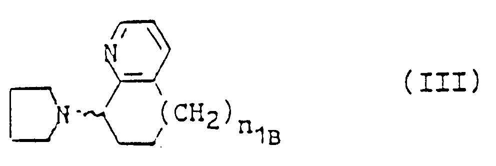 in der n₁B 2 ist, als industrielles Produkt.

12. Verbindung der Formel IV: 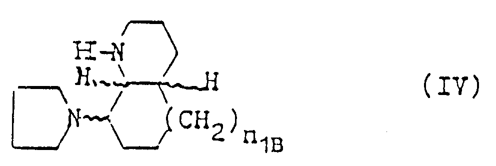 in der n₁B 2 ist, als industrielles Produkt.
